(19) Europäisches Patentamt
European Patent Office
Office européen des brevets

(11) EP 0 799 261 B1

(12) **EUROPEAN PATENT SPECIFICATION**

(45) Date of publication and mention of the grant of the patent:
**26.05.1999 Bulletin 1999/21**

(21) Application number: **95943917.5**

(22) Date of filing: **19.12.1995**

(51) Int Cl.[6]: **C08G 59/02**, C08G 59/34, C07C 41/16, C07C 319/14, C07C 209/18

(86) International application number:
**PCT/US95/16631**

(87) International publication number:
**WO 96/20232 (04.07.1996 Gazette 1996/30)**

(54) **PROCESS FOR PREPARATION OF ALLYL ETHER, THIOETHER AND AMINE COMPOUNDS**

VERFAHREN ZUR HERSTELLUNG VON ALLYL-ETHER-, -THIOETHER- UND -AMIN-VERBINDUNGEN

PROCEDE DE PREPARATION DE COMPOSES D'ETHER, DE THIOETHER ET D'AMINE D'ALLYLE

(84) Designated Contracting States:
**CH DE ES GB IT LI NL**

(30) Priority: **23.12.1994 US 363129**

(43) Date of publication of application:
**08.10.1997 Bulletin 1997/41**

(73) Proprietor: **THE DOW CHEMICAL COMPANY**
**Midland, Michigan 48674 (US)**

(72) Inventors:
- **AU, Andrew, T.**
  **Sugarland, TX 77479 (US)**
- **NAFZIGER, J., Lowell**
  **Lake Jackson, TX 77566 (US)**

(74) Representative: **Raynor, John**
**W.H. Beck, Greener & Co**
**7 Stone Buildings**
**Lincoln's Inn**
**London WC2A 3SZ (GB)**

(56) References cited:
**EP-A- 0 370 946**
**US-A- 4 507 492**
**US-A- 4 740 330**

- **JOURNAL OF ORGANOMETALLIC CHEMISTRY, vol. 326, 1987, pages C23-C28, XP002002520 MUZART J. ET AL: "Palladium complexes -KF/alumina catalysed exchange of allylic groups of esters with phenols" cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 799 261 B1

## Description

The present invention pertains to a process for the preparation of allyl ether, thioether or amine compounds.

Allyl ether, thioether and amine compounds are known to be useful as reactive diluents, as unsaturated monomers or resins, and as intermediates for making epoxy resins and other downstream products. For instance, EPO Publication 0 368 421A2 (16-May-90) has proposed to make epoxy resins by:

(1) reacting allyl halide with a polyhydric phenol to make the poly(allyl ether) of the polyhydric phenol; and
(2) oxidizing the allyl groups in the poly(allyl ether) to make the poly(glycidyl ether) of the polyhydric phenol.

Commonly known processes to make allyl ethers are undesirable because they suffer from low yields or because they produce halogenated waste products or because they need reagents which are not readily available. For example:

(1) Japanese Kokai Hei 5-306246 (published 19-Nov-93) teaches to react an allyl alcohol with a polyhydric phenol in the presence of a palladium or platinum catalyst and an organophosphorus compound. This process uses no halogenated materials, but suffers from very poor yields and poor catalyst efficiency.
(2) Great Britain Patent Specification 1,200,730 (published 29-Jul-70) teaches the reaction of a nucleophilic reagent with a vinyl- or allyl-carboxylate in the presence of a Group VIII metal catalyst. The reference suggests that phenols, thiophenols and polyamines may be nucleophilic reagents, but the only actual examples use inorganic and organic acids.
(3) Woo, U.S. Patent 4,507,492 (March 26, 1985) teaches to react phenols, thiophenols and arylamines with allyl carbonates in the presence of a molybdenum, tungsten or Group VIII metal and triphenyl phosphine to make allyl ethers. This process uses allyl carbonates, which are not readily available.
(4) Takahashi et al., "Palladium-Catalyzed Exchange of Allylic Groups of Ethers and Esters With Active Hydrogen Compounds II," Vol. 45 J. Chem. Soc. - Japan 230, 234 (1972) (Table 5) reports that allyl acetate reacts with phenol in the presence of palladium dichloride, triphenylphosphine and a catalytic amount of sodium phenate at 85°C for 24 hours to yield 14 percent phenyl allyl ether.
(5) J. Muzart et al., Palladium Complexes-KF/Alumina' Catalyzed Exchange of Allylic Groups of Esters With Phenol" Vol. 326 J. Organometallic Chem. C23, C24 (Table 1) (1987) reports that allyl acetate reacts with phenol in the presence of palladium complex and an alumina/KF support at room temperature over 24 hours to yield 84 percent phenyl allyl ether. The catalyst turnover rate of the reaction was 1.

None of these reactions produced a poly(allyl ether) in yields or catalyst efficiencies high enough to form a desirable step for commercially-manufacturing epoxy resins or other downstream intermediates.

What is needed is a practical, high-yield process to make poly(allyl ether) compounds without use of halogenated intermediates.

The present invention is a process to make an allyl ether, allyl thioether or allyl amine comprising the step of reacting an active-hydrogen-containing compound containing at least one hydroxyl, thiol or primary or secondary amine group with a compound containing an allyl carboxylate group in the presence of a transition metal catalyst, a complexing agent and a base; characterized in that the quantity of base is at least 0.8 equivalents per equivalent of active-hydrogen-containing compound.

Using the present invention, the production of the epoxy-containing compounds can be accomplished efficiently in high yields in three steps starting from the basic raw material, propylene; whereas the prior art process involves four or five steps starting from the basic raw material propylene. The three-step process is summarized as follows:

1) Propylene + oxygen + acetic acid + catalyst--> allyl acetate;
2) Allyl acetate + active-hydrogen-containing compound such as a bisphenol → diallyl derivative of active-hydrogen-containing compound; and
3) Diallyl derivative of active-hydrogen-containing compound + peroxy oxidant → diglycidyl derivative of active-hydrogen-containing compound.

Although acetic acid is illustrated above to prepare allyl acetate which is employed as the allylating agent, it is understood that other acids and allylating agents as enumerated elsewhere herein can be employed in the process of the present invention.

The term "hydrocarbyl" as employed herein means any aliphatic, cycloaliphatic, aromatic, aryl substituted aliphatic or cycloaliphatic, or aliphatic or cycloaliphatic substituted aromatic group. Likewise, the term "hydrocarbyloxy"

means a hydrocarbyl group having an oxygen linkage between it and the carbon atom to which it is attached. The term "divalent hydrocarbyl group" refers to the aforementioned hydrocarbyl groups minus an additional hydrogen atom.

The term "catalyst efficiency" or "turnover rate" is defined by the following equation:

$$\text{Catalyst Efficiency or Turnover Rate} = \frac{\text{(Equivalents of Active Hydrogen Converted Formed)}}{\text{(Mole of Catalyst)/Hour}}$$

Allyl Reagent

The allyl reagent is an allyl carboxylate and most preferably is allyl acetate.

The allyl reagent is preferably employed in amounts such that the equivalent ratio of allyl reagent to active-hydrogen atom contained in the active-hydrogen-containing compound is from 0.1:1 to 500:1, preferably from 0.5:1 to 50:1 and more preferably from 1:1 to 5:1. At ratios significantly above 1:1, the allyl reagent performs as a solvent and as a reactant.

At ratios below 0.1:1, the conversion to the desired product is very low and the excess active-hydrogen-containing reactant must be recovered and recycled in the process.

The allyl carboxylates from which the allyl derivatives of active-hydrogen-containing compounds can be prepared include, but are not limited to, for example, those represented by the following Formula I or any combination of any two or more such allyl carboxylates:

**Formula I**

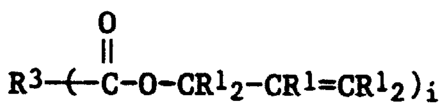

wherein each $R^1$ is preferably hydrogen or an alkyl group having from 1 to 12 carbon atoms which may optionally be linked to another $R^1$ group to form a ring structure, $R^3$ is hydrogen or an alkyl, cycloalkyl or aryl group having from 1 to 10 carbon atoms and "i" equals on average 1 to 3. Preferably, the allyl carboxylate is allyl formate, allyl acetate, allyl propionate, allyl benzoate, and diallyl carboxylates such as oxalate, glutarate, succinate, or any combination thereof. Most preferred as the allyl carboxylate is allyl acetate.

Preparation of Allyl Carboxylates

Certain useful allyl carboxylates, such as allyl acetate, are commercially available. Allyl carboxylates can be prepared by the oxidation of propylene in the presence of oxygen, a carboxylic acid (such as formic acid, acetic acid or propionic acid) and a palladium catalyst as described in U.S. Patent 3,970,713 which is incorporated herein by reference. The reaction is preferably conducted at temperatures of from 50°C to 250°C. In the reaction, from 5 to 20 moles of water per mole of acid, from 1 to 5 moles of oxygen per mole of acid and from 4 to 40 moles of propylene per mole of oxygen are preferably employed. This reaction proceeds readily in one step from the propylene to the allyl carboxylate without the use or production of halogenated materials.

Preparation of the Allyl Derivative of Active-Hydrogen-Containing Compounds

The allyl ether, thioether or amine is prepared by reacting the allyl carboxylate compound with an active-hydrogen-containing compound in the presence of a catalyst, at least one complexing agent for the catalyst and at least one base compound, and optionally but preferably in the presence of one or more suitable solvents.

The reaction is preferably conducted at a temperature of from 0°C to 200°C, more preferably from 25°C to 150°C, more highly preferably from 50°C to 110°C, and most preferably more than 60°C at a pressure sufficient to keep the reaction mixture in liquid form. The reaction time to completion is preferably from 0.1 to 72 hours, more preferably from 0.1 to 48 hours, and most preferably from 0.1 to 24 hours. Optimum temperature and times for particular reactants will vary depending upon the reactivity of the particular reactants, solvents and catalysts employed; however, such optimum temperatures and times can readily be determined in the laboratory with minimum effort.

At temperatures below 0°C, the reaction proceeds at a slower rate and costs for cooling are incurred.

At temperatures above 200°C, costs for providing the increased temperature are incurred.

Particular pressures are not deemed to be determinative of any appreciative effects on the process; however, pressures should be employed which preferably will keep the reaction in the liquid phase.

Suitable allyl ethers, thioethers or amines which can be prepared in the process of the present invention include but are not limited to, for example, those represented by any one of the following Formulas II to IX:

Formula II $R^2\text{-}(Z)_a$

Formula III

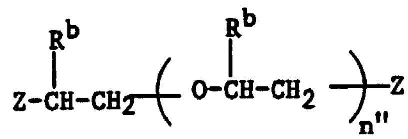

Formula IV

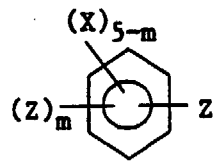

Formula V

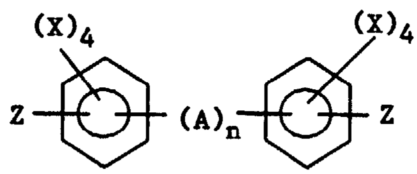

Formula VI

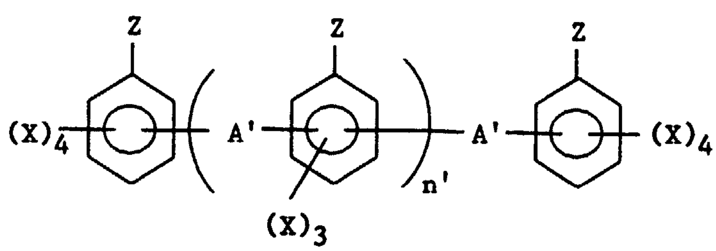

Formula VII

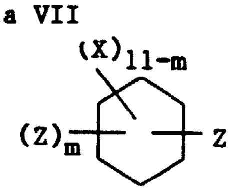

Formula VIII

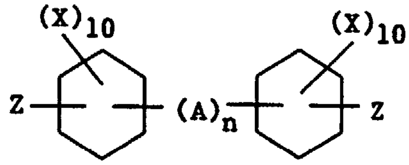

Formula IX

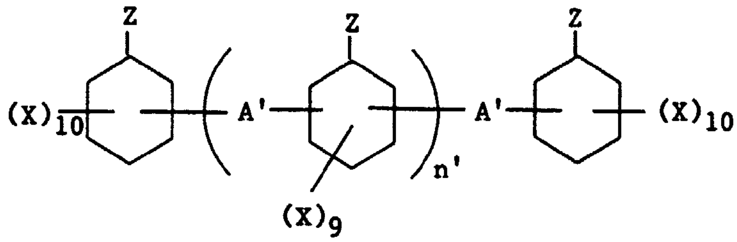

wherein each A is preferably independently a divalent hydrocarbyl group having from 1 to 20, preferably from 1 to 10, and more preferably from 1 to 3 carbon atoms, -O-, -S-, -S-S-, -SO- or $-SO_2-$, -CO-, -COO-, -C=CR-, -C-O-C-, $CHX^a$ ($X^a$ is a halogen or cyanide or a similar inert group); A' is a divalent hydrocarbyl group having from 1 to 12, preferably from 1 to 4, and more preferably from 1 to 2 carbon atoms; each X is independently a hydrogen, a monovalent hydrocarbyl or hydrocarbyloxy group having from 1 to 20, preferably from 1 to 10, and more preferably from 1 to 4 carbon atoms, a halogen atom (preferably chlorine or bromine), or a $-NO_2$ group; each Z is preferably independently a

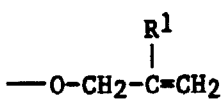 , 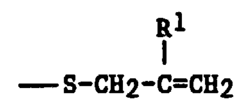 , 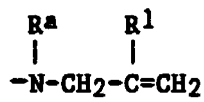

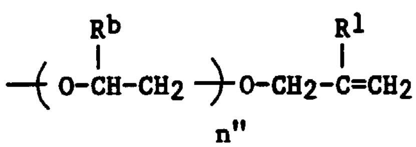 , 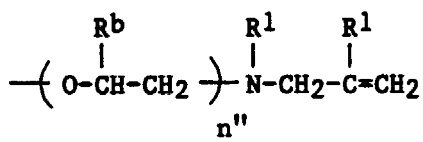 ,

group; $R^a$ is hydrogen, an alkyl group having from 1 to 4 carbon atoms or a $-CR1_2-C(R^1)=CR^1_2$ group; $R^b$ is hydrogen, an alkyl group or a hydrocarbyloxy group having from 1 to 6 carbon atoms; $R^1$ is preferably hydrogen or an alkyl group having from 1 to 4 carbon atoms; $R^2$ is a monovalent or polyvalent aliphatic, cycloaliphatic or aromatic hydrocarbon group having from 2 to 20, preferably from 3 to 10, and more preferably from 4 to 8 carbon atoms; "m" has a value of zero to 3; "n" has a value of zero or 1; "n'" preferably has a value from 0.01 to 10, more preferably from 0.5 to 6, most preferably from 1 to 4; 'n"' has preferably a value from zero to 50, more preferably from zero to 20 and most preferably from zero to 6; and a is preferably 1 to 10, more preferably 2 to 6 and most preferably 2 to 4.

Active-Hydrogen-Containing Compounds

The active-hydrogen-containing compound used in the present invention contains at least one hydroxyl group, thiol group, or primary or secondary amine group per molecule. It more preferably contains on average more than one such group per molecule and most preferably contains on average at least 1.8 such groups per molecule. It preferably contains no more than 10 such groups per molecule. It most preferably contains hydroxyl groups. Examples of suitable

active-hydrogen-containing compounds include: mono, di- or multi-functional aliphatic, cycloaliphatic or aromatic hydroxyl-containing compounds; thiol-containing compounds or; primary and secondary amine-containing compounds; or any combination of such compounds. Particularly suitable examples of active-hydrogen-containing compounds include those represented by any one of Formulas II, III, IV, V, VI, VII, VIII, or IX or any combination of any two or more such active-hydrogen-containing compounds wherein A, A', $R^a$, $R^b$, $R^1$, $R^2$, X, m, n, n' and n" are as defined above and Z is -OH, -SH, $-NH_2$, or $-NH(R^4)$, wherein $R^4$ is hydrogen or a saturated or unsaturated aliphatic group having from 1 to 20, preferably from 1 to 10, and more preferably from 1 to 6 carbon atoms.

Hydroxyl-Containing Compounds Which Are Useful as Active-Hydrogen-Containing Compounds

The hydroxyl-containing compounds are preferably aliphatic, cycloaliphatic or aromatic compounds.

Particularly suitable aromatic hydroxyl-containing compounds include, for example, phenol, cresol, catechol, resorcinol, bisphenol A (p,p'-isopropylidene diphenol), bisphenol F (p,p'-methylene diphenol), bisphenol K (p,p'-diphenolcarbonyl), dihydroxymethylstilbene, dihydroxybiphenyl, dihydroxynaphthalene, bis(hydroxyphenyl)fluorene, phenol-aldehyde novolac resins, alkyl substituted phenol' -aldehyde novolac resins, or any combination thereof. Preferred aromatic hydroxyl-containing compounds include, for example, bisphenol A, bisphenol F, dihydroxymethylstilbene, phenol-formaldehyde novolac resins, cresol-formaldehyde novolac resins or any combination of any two or more of such compounds thereof.

The active-hydrogen-containing compound is preferably a bisphenol, and is most preferably bisphenol A.

Examples of suitable aliphatic hydroxyl-containing compounds include, for example, propanol, butanol, hexanol, octanol, butanediol, hexanediol, octanediol, polyoxyalkylene glycols having a weight average molecular weight of from 106 to 50,000, preferably from 106 to 5,000 and more preferably from 106 to 2500, or any combination thereof.

Examples of particularly suitable cycloaliphatic hydroxyl-containing compounds include, for example, cyclopentanol, cyclohexanol, methylcyclohexanol, dihydroxycyclohexane, norbornanediol, cyclohexanedimethylol, dicyclopentadienediol, vinylcyclohexene derived diols, or any combination thereof.

Thiol Compounds Which Are Useful as Active-Hydrogen-Containing Compounds

Examples of particularly suitable thiol compounds include, for example, thiophenol, bisthiophenol, thiobisphenol A, thiophenol-formaldehyde novolac resins, thiocresol-formaldehyde novolac resins, thionaphthol, or any combination of any two or more compounds thereof.

Primary and/or Secondary Amine-Containing Compounds Which Are Useful as Active-Hydrogen-Containing Compounds

Examples of particularly suitable primary or secondary amine-containing compounds include aryl amines, such as diaminobenzene, aniline, N-methyl aniline, methylene dianiline, hydroxyaniline, bis-aniline, diaminotoluene, amino-naphthylene or any combination thereof.

Base Compounds

The present invention uses at least about a stoichiometric amount of base in relation to the active-hydrogen-containing compound. Suitable base compounds include, for example, alkali and alkaline earth metal bicarbonates, carbonates, phosphates, citrates, acetates, phenoxides, hydroxides, hydrides or alkoxides, such as, for example, sodium carbonate, potassium carbonate, lithium carbonate, calcium carbonate, sodium hydroxide, potassium hydroxide, lithium hydroxide, calcium hydroxide, sodium bicarbonate, potassium bicarbonate, lithium bicarbonate, calcium bicarbonate, or any combination of any two or more such base compounds. Particularly suitable base compounds include, for example, potassium carbonate, sodium carbonate, sodium bicarbonate, potassium hydroxide, sodium hydroxide, or any combination of any two or more such base compounds. Preferred base compounds include, sodium carbonate, sodium bicarbonate and sodium hydroxide.

Suitable resinous bases include, for example, the hydroxide or carbonate quaternary ammonium or amino form of ion exchange resins, particularly styrene/divinyl benzene-based ion exchange resins.

The base compounds are employed in amounts of from 0.8 to 10, preferably from 1 to 2.0 and more preferably from 1 to 1.2 equivalents of base per active hydrogen of the material to be allylated.

Buffering Agents

Strong base compounds, such as hydroxides or alkoxides, are preferably modified with buffering agents such

as, for example, alkali or alkaline earth metal bicarbonates, alkali or alkaline earth metal carbonates, carbon dioxide, alkali or alkaline earth metal hydrogen phosphates, or alkali or alkaline earth metal borates. For example, sodium carbonate may be formed *in situ* using sodium hydroxide and carbon dioxide. The buffering agents are preferably employed in amounts such that the pH of the aqueous portion, if any, in the reaction mixture is from 7 to 13, preferably from 8 to 12 and more preferably from 9 to 11. As an alternative method to control pH, unbuffered bases may be added continuously or in stages over a period of time.

Catalysts

The reaction is run in the presence of a transition metal catalyst. Suitable catalysts preferably contain, for example, rhodium, ruthenium, rhenium, palladium, iridium, tungsten, molybdenum, chromium, cobalt, platinum, nickel, copper, osmium, iron, either in an unoxidized state as the free metal or a complex, or in an oxidized state as their salts such as carboxylates, halides, oxides, nitrates, or sulfates. The preferred catalysts contain palladium, platinum, molybdenum or nickel. The most preferred catalysts contain palladium. Salts are preferably acetate, chloride, nitrate or sulfate salts.

When the catalyst is employed in its free metal state it is preferably supported on a support material such as, for example, carbon, charcoal, activated charcoal, silica, alumina, organosol gel, zeolite, clay or any combination of any two or more of such support materials.

When the catalyst is in the form of a salt, it is preferably not supported.

Homogeneous catalysts are preferably employed in amounts of from 0.005 to 200, highly preferably from 0.01 to 50, more highly preferably from 0.05 to 10 and most preferably from 0.1 to 2 mmoles per equivalent of active-hydrogen atom contained in the active-hydrogen-containing compound.

The supported catalyst preferably contains from 0.1 to 80, more preferably from 0.5 to 50, and most preferably from 0.5 to 10 percent by weight catalyst; and from 99.9 to 20, preferably from 99.5 to 50, and more preferably from 99.5 to 90 percent by weight support material. The supported catalyst preferably contains at least 0.005 mmoles of transition metal per equivalent of active hydrogen, more preferably at least 0.01 mmoles, more highly preferably at least 0.05 mmoles and most preferably at least 0.1 mmoles. The maximum concentration is not critical.

When the catalyst is employed as a supported, heterogeneous catalyst, the reaction can optionally be conducted in a fixed bed or suspended in the liquid reaction mixture.

Complexing Agents

Complexing agents are preferably employed to serve as ligands to stabilize and to enhance the activity of the metal catalyst. The catalyst can be complexed with the complexing agent prior to addition to the reaction mixture or the catalyst complex can be formed *in situ* by adding the catalyst and the complexing agent separately to the reaction mixture.

Suitable complexing agents include, for example, organic mono- or diphosphines, organic phosphites, organic stibines, oximes, organic arsines, diamines, and dicarbonyl compounds. Particularly suitable complexing agents include, for example, triphenyl phosphine, tris-tolyl phosphines, diphenylmethyl phosphine, or diphenylphosphino ethane. The preferred complexing agents include, tris-tolyl phosphine, triphenyl phosphine and diphenylphosphino ethane with triphenyl phosphine being most preferred. The complexing ligand may also be supported on a resinous backbone.

Aqueous soluble complexing agents such as sulfonated triphenylphosphine may also be used. This type of ligand will have the advantage of being water soluble and easily washed out/separated from the organic product layer.

The complexing agents are preferably employed in amounts of from 10 to 1000, more preferably from 50 to 500, and most preferably from 200 to 400 moles per 100 moles of the catalyst excluding any support material.

Solvents

Suitable solvents which can optionally, but preferably, be employed in the process of the present invention include, for example, water, excess amounts of allyl carboxylate, oxygenated hydrocarbons (such as akohols, ethers, glycols, glycol ethers, esters and ketones). Other solvents include nitroalkanes, cyanoalkanes, alkyl sulfoxides, amides, aromatic and aliphatic hydrocarbons, halogenated hydrocarbons or any combination of any two or more such solvents can also be used. Particularly suitable solvents include, for example, water, allyl acetate, toluene, xylene, methylene chloride, 1,2-dichloroethane, acetonitrile, acetone, methyl ethyl ketone, ethyl acetate, dimethylformamide, dimethylsulfoxide, nitromethane, tetrahydrofuran, ethanol, isopropanol, t-butanol, t-amyl alcohol, glymes or any combination thereof. The preferred solvents are water, allyl acetate, isopropanol, t-butanol, t-amyl alcohol, acetone, acetonitrile, toluene, 1,2-dichloroethane, glycols and glycol ethers or any combination of any two or more such solvents. A more highly preferred system is a biphasic solvent system containing an aqueous phase and an organic phase. The organic

phase preferably uses excess allyl carboxylate compound as a solvent. The mixture is preferably mixed vigorously to bring the two phases into intimate contact with each other. The weight ratio of aqueous to organic phase is preferably 9:1 to 1:9.

The solvents are preferably employed in amounts of from zero to 100, more preferably from 0 to 20, and most preferably from 0 to 10 parts by weight based upon the weight of active-hydrogen-containing component.

One preferred use for the allyl ether, allyl thioether or allyl amine compounds is to convert it to an epoxy compound by the methods known in the art for epoxidizing ethylenic or olefinic double bonds with peroxygen compounds. Suitable methods include those disclosed in <u>Epoxy Resins Chemistry and Technology,</u> 2nd Ed., 1988, Clayton A. May, Marcel Dekker, Inc, Chapter 2, part III, pp. 15-46; Buchler et al., U.S. Patent 4,973,718 (November 27, 1990); Schmidt et al., U.S. Patent 4,845,252 (July 4, 1989); and Kim, U.S. Patent 4,418,203 (November 29, 1993).

For example, the epoxidation may be conducted by reacting the allyl ethers with a peroxygen compound such as peracetic acid or hydrogen peroxide in the presence of catalysts such as $Na_2WO_4/H_3PO_4$/quaternary ammonium salt such as, for example, trioctyl methyl ammonium nitrate. Both techniques are well-known in the art.

The hydrogen peroxide epoxidation is conducted at temperatures of from 0°C to 100°C, preferably from 20°C to 60°C and more preferably from 35°C to 50°C.

The peracetic acid epoxidation is conducted at temperatures of from 20°C to 200°C, preferably from 40°C to 100°C and more preferably from 40°C to 80°C.

A highly preferred summary of the entire three-step process from propylene to epoxy resin is as follows:

1) Propylene + oxygen + acetic acid + catalyst → allyl acetate;
2) Allyl acetate + active-hydrogen-containing compound such as bisphenol A → diallyl derivative of active-hydrogen-containing compound; and
3) Diallyl derivative of active-hydrogen-containing compound + peroxy oxidant → diglycidyl derivative of active-hydrogen-containing compound.

The following examples are illustrative of the invention and should not be interpreted as limiting it in any way. Unless stated otherwise all parts and percentages are given by weight.

In the following examples, the amount of total halide is determined by digesting the sample with caustic (NaOH) and titrating with silver nitrate.

In the following examples, the epoxide equivalent weight (EEW) is determined by titration with an acid.

<u>Example 1</u>

<u>A. Preparation of Bisphenol A Diallyl Ether From Bisphenol A and Allyl Acetate Using a Heterogeneous Palladium Catalyst.</u>

To a mixture of 11.4 g (0.05 mole) of bisphenol A, 0.1 g of 5 percent palladium (0.0469 mmole) on charcoal, 0.1 g (0.38 mmole) of triphenyl phosphine, and 14.5 g (0.105 mole) of potassium carbonate in 30 mL of isopropanol under a nitrogen atmosphere was added 11.0 g (1.1 equiv.) of allyl acetate and the mixture stirred at 85°C. After 4 hours, both thin layer chromatography and high performance liquid chromatography showed that the reaction was practically completed and showed only one product peak. The catalyst turnover rate was calculated to be 533 equivalent of active hydrogen/mole of catalyst/hour.

To the mixture prepared above, 30 mL of water and 100 mL of ethyl acetate were added. The mixture was shaken well. The mixture was filtered and the organic layer was separated, dried and concentrated in vacuo at 2 mm Hg and 50°C to yield 15.3 g of a colorless oil representing a yield of 99.4 percent based on bisphenol A. Both infrared (IR; with no OH absorption) and nuclear magnetic resonance (NMR; appearance of an appropriate quantity of corresponding allylic protons) spectra were consistent with the desired product of bisphenol A diallyl ether and identical to a known sample of diallyl ether of bisphenol A which was derived from bisphenol A and allyl chloride.

<u>B. Epoxidation of Bisphenol A Diallyl Ether.</u>

To a solution of 12.5 g of diallyl ether of bisphenol A (40.6 mmole) and 3.2 g (7.6 mmole) of trioctylmethylammonium nitrate in 30 mL of toluene was added an aqueous solution of 2.6 g of sodium tungstate (8.8 mmole), 1.6 g of phosphoric acid (16.3 mmole) and 16 g (about 0.15 mole) of 30 percent hydrogen peroxide in 32 mL of water (final pH of 2.05). The mixture was stirred at 40°C and high performance liquid chromatography was used to monitor the progress of the reaction. After 4 days, the reaction was stopped when total disappearance of the allyl ether and the formation of a new product peak were observed. The aqueous layer was separated and extracted twice with 20 mL portions of toluene. The organic reaction mixture was combined with the extracts, washed twice with 30 mL portions of water, and

dried over magnesium sulfate and then concentrated in vacuo resulting in 17.0 g of a brown oil.

This crude brown oil was passed through a column of 250 g of silica gel packed in 20 percent of acetone in hexane. Elution of this column with a gradient of 20 to 50 percent acetone in hexane yielded 9.5 g of a light yellow oil. Spectral data (IR and NMR; NMR: absence of olefinic protons and appearance of epoxide protons) were consistent with the desired product (the diglycidyl ether of bisphenol A). Furthermore, both thin layer chromatography and high performance liquid chromatography showed identical retention times with a known sample of diglycidyl ether of bisphenol A. Analysis showed that the product obtained contained 26 ppm of total chloride and had an epoxy equivalent weight of 182.

Example 2

Preparation of Bisphenol A Diallyl Ether From Bisphenol A and Allyl Acetate Using Homogeneous Palladium Catalyst

To a glass reactor equipped with glass baffles under a nitrogen atmosphere was added a solution of 35.0 g (0.875 mol) of sodium hydroxide in 300 mL of water. Then 40 g of dry ice was gradually added until a pH of 10.5 was obtained. Then 45.6 g (0.2 mole) of bisphenol A was added. After stirring for 20 minutes, the mixture was heated to 85°C and 80.0 g (0.8 mole) of allyl acetate, 0.028 g (0.125 mmole) of palladium acetate and 0.162 g (0.617 mmole) of triphenyl phosphine were added. After stirring rapidly (about 600 rpm) for 15 minutes, the reaction was >99 percent complete and the high performance liquid chromatogram showed cleanly one product peak. The stirring was stopped and the organic layer was separated and concentrated in vacuo to yield 58.61 g of desired product. The yield was 95.2 percent. The catalyst turnover rate was calculated to be 12,800/hour.

Repeating the above example using a stirring rate of 60 rpm instead of 600 rpm the conversion was only 14.4 percent after 15 minutes.

Example 3

A. Preparation of Allyl Phenolic Novolac

To a mixture of 6.2 g (20 mmole) of cresol novolac (average molecular weight = 310, hydroxy equivalent weight = 124, average functionality = 2.5), 8.0 g (57.9 mmole) of potassium carbonate, 50 mg of 5 percent palladium (0.024 mmole) on charcoal, and 20 mg (0.08 mmole) of triphenyl phosphine in 25 mL of isopropanol, was slowly added 6.0 g (60 mmole) of allyl acetate. After the resultant mixture was stirred at 85°C for 8 hours, high performance liquid chromatography showed that the reaction was complete. Water (30 mL) and toluene (30 mL) were added to the mixture. After extracting, the toluene layer was separated and the aqueous layer was again extracted with another 30 mL of toluene. After separation, the toluene extracts were combined, dried over magnesium sulfate and concentrated in vacuo to a viscous pale yellow oil (7.8 g, 95 percent yield). Spectral data of this material were consistent with the desired allylated product. The NMR spectrum showed the presence of the vinylic-H and the allylic-hydrogens while the IR spectrum showed the absence of OH absorption.

B. Epoxidation of Allyl Phenol Novolac

An aqueous solution containing 2.65 g (8 mmole) of sodium tungstate dihydrate, 1.6 g (16 mmole) of phosphoric acid and 8.0 g (0.165 mole) of 70 percent hydrogen peroxide in 28 mL of water (mixture pH = 2.05) was added to a 24 mL toluene solution containing 12.6 g (0.1 equiv.) of allylated cresol novolac prepared in A above and 1.6 g (4.5 mmole) of trioctylmethylammonium nitrate. The mixture was stirred at 45°C for 28 hours and was stopped when almost all starting material had disappeared. The mixture was cooled and the organic layer separated and washed 3 times with 50 mL of water. It was then dried over magnesium sulfate, filtered and concentrated in vacuo to give 13.6 g of a viscous amber liquid.

To remove the ammonium nitrate from the crude product, the material was extracted with 5 mL of methanol by warming up with stirring, cooling to room temperature and decanting the extracting methanol. The process was repeated four times. The product was then concentrated with roto-evaporator (<100°C/2 mm Hg), yielding 9.1 g of desired cresol epoxy novolac {analysis: total organic Cl = 1 ppm, percent epoxide = 19.12 percent (EEW = 224.9)}.

Example 4

Allylation of Benzyl Alcohol

A mixture of benzyl alcohol (10.8 g, 0.1 mole), allyl acetate (10.5 g, 0.105 mole) and potassium carbonate

(15 g, 0.109 mole) in 15 mL of water was stirred under nitrogen for 15 minutes. Then 10 mg (0.045 mmole) of palladium acetate and 30 mg (0.115 mmole) of triphenyl phosphine were added and the mixture was stirred at 85°C. After 48 hours, the reaction was 65 percent completed, and the reaction rate became quite sluggish. After cooling, 50 mL of water and 50 mL of toluene were added for extraction. The organic extract was separated and washed twice with 30 mL of water, dried over magnesium sulfate and concentrated in vacuo yielding 14.2 g. This light brown liquid was distilled and collected at 71°C to 74°C/13 mm Hg, yielding 8.5 g of a colorless liquid (NMR and IR spectra were consistent with the desired benzyl allyl ether).

B. Epoxidation of Benzyl Allyl Ether

An aqueous solution containing 1.32 g (0.004 mole) of sodium tungstate dihydrate, 1.0 g (0.012 mole) of phosphoric acid and 12.5 g (0.11 mole) of 30 percent hydrogen peroxide in 18 mL of water (mixture pH = 1.98) was added to a 24 mL toluene solution containing 8.5 g (0.574 mole) of benzyl allyl ether and 1.0 g (0.0023 mole) trioctylmethylammonium nitrate. The mixture was stirred at 45°C for 36 hours and was stopped when almost all starting material had disappeared. The mixture was cooled and the organic layer was separated and washed three times with 50 mL of water. It was then dried over magnesium sulfate, filtered and concentrated in vacuo to give 12.2 g of a viscous amber liquid.

To remove the ammonium nitrate from the crude product, the material was chromatographed over 200 g of silica gel packed in 10 percent acetone in hexane. Elution of this column with a gradient of 10 to 20 percent acetone/hexane yielded 8.2 g of material analyzed to have total organic chloride of 199 ppm and an EEW of 164.

Example 5

A. Allylation of Aniline

A mixture of aniline (2.4 g, 25 mmole) potassium carbonate (8.0 g, 60 mmole), 6.0 g (60 mmole) of allyl acetate, 50 mg of palladium (0.011 mmole) on charcoal and 20 mg (0.08 mmole) of triphenyl phosphine in 25 mL of isopropanol was stirred for 22 hours. When gas chromatography showed the absence of the starting material and the appearance of a new peak indicating that the reaction was complete, water (20 mL) and ethyl acetate (40 mL) were added for extraction. The extract was dried and concentrated in vacuo to yield 3.5 g (78.7 percent yield) of an amber oil whose NMR spectrum was consistent with the structure of diallyl aniline.

Examples 6-11 - Allylation of Phenol

Several different mixtures of phenol and allyl acetate were reacted together under inert atmosphere in 3-neck, round-bottom flasks fitted with a gas inlet, reflux condenser and a thermometer equipped with a temperature controller. The reactor contents were heated to about 85°C using an infrared lamp and stirred using a magnetic stirrer. The reagents, the reaction time, and the ratio of allyl phenyl ether to phenol in the product mixture are shown in Table I. Analyses were obtained using gas chromatography.

TABLE I

| Example | 6 | 7 | 8 | 9 | 10 | 11 | A* |
|---|---|---|---|---|---|---|---|
| Phenol (moles) | 0.05 | 0.02 | 0.1 | 0.1 | 0.05 | 0.02 | 0.02 |
| Allyl Ace. (moles) | 0.06 | 0.022 | 0.12 | 0.12 | 0.06 | 0.022 | 0.022 |
| Solvent/mL | IPA/15 | IPA/8 Water/2 | Water/16 | Water/16 | Water/10 | IPA/100 | IPA/100 |
| Base | $K_2CO_3$ | $K_2CO_3$ | $NaOH/CO_2$ | $NaOH/CO_2$ | $K_2CO_3$ | $K_2CO_3$ | KOH |
| Moles of Base | 0.054 | 0.023 | 0.11 | 0.11 | 0.054 | 0.024 | 0.011 |
| Catalyst | Pd/C | Pd/C | Pd/C | Pd/C | Pd/C | $PdCl_2$ | Pd/C |
| mg of Catalyst | 50 | 50 | 50 | 50 | 50 | 50 | 50 |
| mg of TPP | 30 | 50 | 30 | 30 | 50 | 50 | 50 |
| Time (hr) | 3 | 4 | 7 | 7 | 12 | 4 | 4 |
| Ratio: PhOH/APhE | 1/98 | 1/99 | 10/90 | 28/72 | 14/86 | 15/85 | 55/7 |
| Comments | | | pH=10.6 | pH=11.6 | He atmo-sphere | | |

Solvents: IPA = isopropanol
Catalysts: Pd/C = palladium on carbon
Ligands: TPP = triphenylphosphine
Products: PhOH = phenol; APhE = allyl phenyl ether
* - not an example of the invention

Examples 12-18 - Allylation of Bisphenol A

Several different mixtures of bisphenol A and allyl acetate were reacted together under inert atmosphere in 3-neck, round-bottom flasks fitted with a gas inlet, reflux condenser and a thermometer equipped with a temperature controller. The reactor contents were heated using an infrared lamp to 85°C and stirred using a magnetic stirrer. The reagents, the reaction time, conversion and the ratio of diallyl ether of bisphenol A to monoallyl ether of bisphenol A in the product mixture are shown in Table II. Analyses were obtained using high performance liquid chromatography.

TABLE II

| Example | 12 | 13 | 14 | 15 | 16 | 17 | 18 |
|---|---|---|---|---|---|---|---|
| BPA (moles) | 0.75 | 0.75 | 0.025 | 0.025 | 0.01 | 0.01 | 0.01 |
| Allyl Ace. (moles) | 1.8 | 1.8 | 0.06 | 0.06 | 0.06 | 0.06 | 0.06 |
| mL of Water | 0 | 250 | 8 | 8 | 5 | 8 | 5 |
| Base | $K_2CO_3$ | $K_2CO_3$ | $K_2CO_3$ | $K_2CO_3$ | $Na_2CO_3$ | $NaHCO_3$ | $Na_2CO_3$ |
| Moles of Base | 1.8 | 1.8 | 0.058 | 0.058 | 0.03 | 0.03 | 0.015 |
| Catalyst | Pd/C | Pd/C | $Pd/Al_2O_3$ | $Pd/BaSO_4$ | $Pd(Ac)_2$ | $Pd(Ac)_2$ | $Pd(Ac)_2$ |
| mg of Catalyst | 1500 | 1500 | 25 | 25 | 15 | 15 | 15 |
| mg of TPP | 600 | 600 | 25 | 25 | 30 | 30 | 30 |
| Time (hr) | 18 | 9 | 8 | 8 | 2 | 2 | 4 |
| BPA Conversion | 100 | 100 | 98 | 96 | - | - | - |
| Ratio MAE/DAE | 0/100 | 1/99 | 19/79 | 26/70 | 8/90 | 0/100 | 0/100 |

Catalysts: Pd/C = palladium on carbon; Pd/Al2O3 = palladium on alumina; $Pd/BaSO_4$ = palladium on barium sulfate; $Pd(Ac)_2$ = palladium diacetate

Ligands: TPP = triphenylphosphine

Compounds: BPA = bisphenol A; MAE = monoallyl ether of bisphenol A; DAE = diallyl ether of bisphenol A

Examples 19-21

Mixtures containing 60 g (0.6 mole) of allyl acetate, 45 g (0.2 mole) of bisphenol A, 150 mL of organic solvent and 100 mL of water were placed in a glass reactor equipped with glass baffles under nitrogen atmosphere and heated to 80°C. A mixture containing 28 mg of palladium diacetate and 162 mg of triphenylphosphine was added to the reaction mixture. After stirring at 400 rpm for a short time, the temperature was raised to 85°C and a 50 percent aqueous solution of sodium hydroxide was added continuously to the reaction mixture for the time shown in Table III. After the addition was completed, the reaction mixture was analyzed by gas chromatagraphy. The results are shown in Table III:

TABLE III

| Example | 19 | 20 | 21 |
|---|---|---|---|
| Solvents | t-AA | DMEE | t-AA |
| g(mole) of 50% NaOH | 48 (0.6) | 48 (0.6) | 38.4 (0.48) |
| Time (hr) | 2.5 | 2.5 | 2.7 |
| Product Composition (%) | BPA - 0.2 | BPA - 0.23 | BPA - 0.2 |
| | MAE - 0.26 | MAE - 0.35 | MAE - 0.15 |
| | DAE - 98.7 | DAE - 98.8 | DAE - 98.9 |
| | Other - 0.8 | Other - 0.6 | Other - 0.7 |

BPA = bisphenol A; MAE = monoallyl ether of bisphenol A; DAE = diallyl ether of bisphenol A; t-AA = t-amyl alcohol; DMEE = dimethoxyethyl ether

## Claims

1. A process to make an allyl ether, allyl thioether or allyl amine comprising the step of reacting an active-hydrogen-containing compound containing at least one hydroxyl, thiol or amine group with a compound containing an allyl carboxylate group in the presence of a transition metal catalyst, a complexing agent and a base; characterized in that the base contains any one or more of: alkali, alkaline earth or ammonium bicarbonates, carbonates, hydrides, hydroxides, alkoxides or phenoxides and the quantity of base is at least 0.8 equivalents per equivalent of active-hydrogen-containing compound.

2. A process as claimed in Claim 1 wherein the concentration of base is at least one equivalent per equivalent of active-hydrogen-containing compound.

3. A process as claimed in any of the preceding Claims wherein the base is added continuously or in multiple stages.

4. A process as claimed in any of the preceding Claims wherein the active-hydrogen-containing compound contains on average more than 1 active-hydrogen-containing group, which are independently any of hydroxyl, thiol, or amine groups per molecule.

5. A process as claimed in any of the preceding Claims wherein the reaction is carried out at a temperature of more than 60°C.

6. A process as claimed in any of the preceding Claims wherein the reaction is carried out in a mixture which contains an organic phase and an aqueous phase.

7. A process as claimed in any of the preceding Claims wherein the equivalent ratio of allyl carboxylate compound to active-hydrogen-containing compound is at least 1:1.

8. A process as claimed in any of the preceding Claims wherein the catalyst contains from 0.005 to 200 millimoles of palladium per equivalent of active-hydrogen-containing compound, and wherein the complexing aaent is any one or more of: an organic mono- or diphosphine, an organic phosphite, an organic stibine, an oxime, an organic arsine, a diamine or a dicarbonyl compound.

9. A process as claimed in any of the preceding Claims wherein the active-hydrogen-containing compound contains

any one or more of: bisphenol A, bisphenol F, bisphenol K, phenol-novolac, cresol-novolac, hydrocarbon-novolac or trisphenol; and the allyl carboxylate compound is allyl acetate.

**10.** A process as claimed in any of the preceding Claims wherein a bisphenol is reacted with allyl acetate in the presence of an aqueous and an organic phase in the presence of a catalyst which contains palladium and a phosphine complexing agent and in the presence of an alkali or alkaline earth metal bicarbonate, carbonate, hydroxide or alkoxide at a temperature above 60°C.

**11.** A process to make an epoxide compound containing the steps of:

(1) making an allyl ether, allyl thioether or allyl amine compound; and

(2) epoxidizing at least a portion of the double bonds in the allyl ether, allyl thioether or allyl amine compound,

characterized in that Step (1) is a process as claimed in any of the preceding Claims.

**Patentansprüche**

**1.** Verfahren zur Herstellung eines Allylethers, Allylthioethers oder Allylamins, umfassend die Stufe der Umsetzung einer aktiven Wasserstoff enthaltenden Verbindung, die wenigstens eine Hydroxyl-, Thiol- oder Amingruppe enthält, mit einer Verbindung, die eine Allylcarboxylatgruppe enthält, in Anwesenheit eines Übergangsmetallkatalysators, eines Komplexbildners und einer Base, dadurch gekennzeichnet, daß die Base eines oder mehrere enthält von: Alkali-, Erdalkali- oder Ammoniumbikarbonaten, -karbonaten, -hydriden, -hydroxiden, -alkoxiden oder -phenoxiden und die Menge der Base wenigstens 0,8 Äquivalente pro Äquivalent von aktiven Wasserstoff enthaltender Verbindung beträgt.

**2.** Verfahren nach Anspruch 1, bei welchem die Konzentration von Base wenigstens 1 Äquivalente pro Äquivalent von aktiven Wasserstoff enthaltender Verbindung beträgt.

**3.** Verfahren nach einem der vorhergehenden Ansprüche bei welchem die Base kontinuierlich oder in mehreren Stufen zugesetzt wird.

**4.** Verfahren nach einem der vorhergehenden Ansprüche bei welchem die aktiven Wasserstoff enthaltende Verbindung einen Durchschnitt von mehr als 1 aktiven Wasserstoff enthaltender Gruppe, die unabhängig voneinander beliebige Hydroxyl-, Thiol- oder Amingruppen sein können, enthält.

**5.** Verfahren nach einem der vorhergehenden Ansprüche bei welchem die Umsetzung bei einer Temperatur von mehr als 60°C durchgeführt wird.

**6.** Verfahren nach einem der vorhergehenden Ansprüche bei welchem die Umsetzung in einem Gemisch durchgeführt wird, das eine organische Phase und eine wässrige Phase enthält.

**7.** Verfahren nach einem der vorhergehenden Ansprüche bei welchem das Äquivalentverhältnis von Allylcarboxylatverbindung zu aktiven Wasserstoff enthaltender Verbindung wenigstens 1:1 beträgt.

**8.** Verfahren nach einem der vorhergehenden Ansprüche bei welchem der Katalysator von 0,005 bis 200 Millimol Palladium pro Äquivalent von aktiven Wasserstoff enthaltender Verbindung enthält, und bei welchem der Komplexbildner ein beliebiger von einer oder mehreren von: organischer Mono- oder Diphosphin-, einer organischen Phosphit-, einer organischen Stilben-, einer Oxim-, einer organischen Arsin-, einer Diamin- oder einer Dicarbonylverbindung ist.

**9.** Verfahren nach einem der vorhergehenden Ansprüche bei welchem die aktiven Wasserstoff enthaltende Verbindung eine oder mehrere von: Bisphenol-A, Bisphenol-F, Bisphenol-K, Phenol-Novolak, Kresol-Novolak, Kohlenwasserstoff-Novolak oder Trisphenol enthält und die Allylcarboxylatverbindung Allylacetat ist.

**10.** Verfahren nach einem der vorhergehenden Ansprüche bei welchem ein Bisphenol mit Allylacetat in Anwesenheit einer wässrigen und einer organischen Phase in Anwesenheit eines Katalysators, der Palladium enthält, und eines

Phosphin-Komplexbilders und in Anwesenheit eines Alkali- oder Erdalkalimetallbikarbonates, -karbonates, -hydroxids oder -alkoxids bei einer Temperatur oberhalb 60°C umgesetzt wird.

11. Verfahren zur Herstellung einer Epoxidverbindung, umfassend die Stufen von:

(1) Herstellung einer Allylether-, Allylthioether- oder Allylaminverbindung; und
(2) Epoxidieren wenigstens eines Teiles der Doppelbindungen in der Allylether-, Allylthioether- oder Allylaminverbindung,

dadurch gekennzeichnet, daß Stufe (1) ein Verfahren ist, wie es in einem der vorhergehenden Ansprüche beansprucht ist.

**Revendications**

1. Procédé pour fabriquer un éther allylique, un thioéther allylique ou une allylamine, qui comprend l'étape consistant à faire réagir un composé à atome(s) d'hydrogène actif(s) qui contient au moins un groupe hydroxyle, thiol ou amine, avec un composé contenant un groupe carboxylate d'allyle, en présence d'un catalyseur au métal de transition, d'un agent complexant et d'une base ; caractérisé en ce que la base contient un quelconque des bicarbonates, carbonates, hydrures, hydroxydes, alkylates et phénylates de métal alcalin ou alcalino-terreux ou d'ammonium, ou plusieurs d'entre eux, et la quantité de base vaut au moins 0,8 équivalent par équivalent de composé à atome(s) d'hydrogène actif(s).

2. Procédé selon la revendication 1, dans lequel la concentration de la base vaut au moins un équivalent par équivalent de composé à atome(s) d'hydrogène actif(s).

3. Procédé selon l'une quelconque des précédentes revendications, dans lequel on ajoute la base en continu ou en plusieurs étapes.

4. Procédé selon l'une quelconque des précédentes revendications, dans lequel le composé à atome(s) d'hydrogène actif(s) contient en moyenne plus d'un groupe à atome d'hydrogène actif, qui est indépendamment un quelconque des groupes hydroxyle, thiol ou amine, par molécule.

5. Procédé selon l'une quelconque des précédentes revendications, dans lequel on effectue la réaction à une température supérieure à 60 °C.

6. Procédé selon l'une quelconque des précédentes revendications, dans lequel on effectue la réaction dans un mélange qui contient une phase organique et une phase aqueuse.

7. Procédé selon l'une quelconque des précédentes revendications, dans lequel le rapport d'équivalent du composé de type carboxylate d'allyle au composé à atome(s) d'hydrogène actif(s) vaut au moins 1:1.

8. Procédé selon l'une quelconque des précédentes revendications, dans lequel le catalyseur contient de 0,005 à 200 millimoles de palladium par équivalent de composé à atome(s) d'hydrogène actif(s), et dans lequel l'agent complexant est un quelconque des mono- ou diphosphine organique, phosphite organique, stibine organique, oxime, arsine organique, diamine et composé dicarbonylé, ou plusieurs d'entre eux.

9. Procédé selon l'une quelconque des précédentes revendications, dans lequel le composé à atome(s) d'hydrogène actif(s) contient un quelconque des bisphénol A, bisphénol F, bisphénol K, phénol-novolaque, crésol-novolaque, hydrocarbure-novolaque et trisphénol, ou plusieurs d'entre eux ; et le composé de type carboxylate d'allyle est l'acétate d'allyle.

10. Procédé selon l'une quelconque des précédentes revendications, dans lequel on fait réagir un bisphénol avec l'acétate d'allyle en présence d'une phase aqueuse et d'une phase organique, en présence d'un catalyseur qui contient du palladium et d'un agent complexant de type phosphine, et en présence d'un bicarbonate, carbonate, hydroxyde ou alkylate de métal alcalin ou alcalino-terreux à une température supérieure à 60 °C.

11. Procédé pour fabriquer un composé époxyde, qui contient les étapes consistant à :

(1) fabriquer un composé de type éther allylique, thioéther allylique ou allylamine; et à
(2) époxyder au moins une partie des doubles liaisons dans le composé de type éther allylique, thioéther allylique ou allylamine,

caractérisé en ce que l'étape (1) est un procédé selon l'une quelconque des précédentes revendications.